# EUROPÄISCHE PATENTANMELDUNG

(11) **EP 0 736 608 A1**
(43) Veröffentlichungstag der Anmeldung: **09.10.1996**
(21) Anmeldenummer: 95105346.1
(22) Anmeldetag: 08.04.1995
(51) Int. Cl.: C12Q 1/68, C12N 9/12, C12P 19/34

(54) **Verfahren zur spezifischen Vervielfältigung und zum Nachweis von DNA bzw. RNA**

(71) Anmelder: BOEHRINGER MANNHEIM GMBH, 68298 Mannheim (DE)
(72) Erfinder: Frey, Bruno, Dr., D-82377 Penzberg (DE); Kübler, Hildegund, D-82362 Weilheim (DE)

(57) **Zusammenfassung**

Die Erfindung betrifft eine Enzymmischung bestehend aus zwei thermophilen DNA-Polymerasen mit und ohne proofreading-Aktivität und weiterer Hilfssubstanzen für die PCR und deren Verwendung zur Vervielfältigung von einzel- und doppelsträngigen kurzen DNA-Fragmenten. Das Verfahren zur Vervielfältigung und gegebenenfalls zur Markierung der kurzen DNA-Fragmente zeichnet sich insbesondere durch eine Elongationstemperatur von ca. 72 °C und eine Widerstandsfähigkeit gegen hohe Magnesiumkonzentrationen aus.

SEQUENZPROTOKOLL

(1) ALLGEMEINE ANGABEN:

(i) ANMELDER:
(A) NAME: BOEHRINGER MANNHEIM GMBH
(B) STRASSE: Sandhofer Str. 116
(C) ORT: Mannheim
(E) LAND: Deutschland
(F) POSTLEITZAHL: D-68305
(G) TELEFON: 0621/759-3277
(H) TELEFAX: 0621/759-4457

(ii) BEZEICHNUNG DER ERFINDUNG: Verfahren zur spezifischen
Vervielfaeltigung und zum Nachweis von DNA bzw. RNA

(iii) ANZAHL DER SEQUENZEN: 16

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(v) DATEN DER JETZIGEN ANMELDUNG:
ANMELDENUMMER: EP 95105346.1

(2) ANGABEN ZU SEQ ID NO: 1:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 1:

TAAAGGGTCA CCGTGGCTTC 20

(2) ANGABEN ZU SEQ ID NO: 2:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 2:

CGAACCACAT TGGCATCATC 20

(2) ANGABEN ZU SEQ ID NO: 3:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 3:

TGGAAACTTT CCACTTGAT 19

(2) ANGABEN ZU SEQ ID NO: 4:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 4:

GTCCCAAGCA ATGGATCAT 19

(2) ANGABEN ZU SEQ ID NO: 5:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 5:

GGAATTCTAT GGGAGTGCAG GTGGAA 26

(2) ANGABEN ZU SEQ ID NO: 6:

(i) SEQUENZKENNZEICHEN:
(A) LÄNGE: 35 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 6:

GCGGATCCAA GCTTTCATTC CAGTTTTAGA AGCTC 35

(2) ANGABEN ZU SEQ ID NO: 7:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 7:

GCTGCATCAT ATAAGTTGCC 20

(2) ANGABEN ZU SEQ ID NO: 8:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 8:

AAGGCTACAC TGTTAATTTT 20

(2) ANGABEN ZU SEQ ID NO: 9:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 9:

GTCCCAAGCA ATGGATGAT 19

(2) ANGABEN ZU SEQ ID NO: 10:

(i) SEQUENZKENNZEICHEN:
(A) LÄNGE: 19 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 10:

TGGAAACTTT CCACTTGAT 19

(2) ANGABEN ZU SEQ ID NO: 11:

(i) SEQUENZKENNZEICHEN:
(A) LÄNGE: 34 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 11:

GGAAGTACAG CTCAGAGTTC TGCAGCACCC CTGC 34

(2) ANGABEN ZU SEQ ID NO: 12:

(i) SEQUENZKENNZEICHEN:
(A) LÄNGE: 31 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 12:

GATGCGAAAC TGAGGCTGGC TGTACTGTCT C 31

(2) ANGABEN ZU SEQ ID NO: 13:

(i) SEQUENZKENNZEICHEN:
(A) LÄNGE: 31 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 13:

TGTCTCCAGC ACACAGCATG TTGTCGGTGA C 31

(2) ANGABEN ZU SEQ ID NO: 14:

(i) SEQUENZKENNZEICHEN:
(A) LÄNGE: 31 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 14:

CAAAGTCATG CGGCCATCGT TCAGACACAC C 31

(2) ANGABEN ZU SEQ ID NO: 15:

(i) SEQUENZKENNZEICHEN:
(A) LÄNGE: 20 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 15:

AGACAGTACA GCCAGCCTCA 20

(2) ANGABEN ZU SEQ ID NO: 16:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer"

(xi) SEQUENZBESCHREIBUNG: SEQ ID NO: 16:

GACTTCAAAT TTCTGCTCCT C 21

## Beschreibung

Die Erfindung betrifft eine Enzymmischung bzw. deren Verwendung zur spezifischen Vervielfältigung von kurzen Nukleinsäuresequenzen bzw. ein Verfahren zum spezifischen Nachweis solcher Nukleinsäuresequenzen in Gegenwart einer Probe, insbesondere in biologischen Flüssigkeiten.

Die Vermehrung von einzel- bzw. doppelsträngigen Nukleinsäuresequenzen in Gegenwart bestimmter Primer und eines die Polymerisation induzierenden Agenzes, wie z. B. hitzestabile DNA-Polymerasen oder reverse Transkriptasen, finden heutzutage eine breite Anwendung, insbesondere in der klinischen Diagnostik. Das mehrere Cyclen durchlaufende Verfahren ist hinlänglich als Polymerase-Ketten-Reaktion (PCR) bekannt (EP 0 200 362, EP 0 258 017). Die PCR-Reaktion wird üblicherweise mit Hilfe der thermophilen DNA-Polymerase aus Thermus aquaticus, sogenannter Taq-Polymerase durchgeführt. Dieses sogenannte klassische PCR-Verfahren zeigt jedoch bei geringen Mengen einer Nukleinsäuresequenz, wie z. B. genomischer DNA, nur eine niedrige Effizienz bzw. führt in vielen Fällen zu keiner spezifischen Vervielfältigung. Dies kann oft selbst nicht durch eine höhere Cyclenzahl ausgeglichen werden, da sich dadurch der Anteil fehlerhaft abgelesener Sequenzen beträchtlich erhöht.

Zudem treten entsprechende Phänomene verstärkt bei längeren DNA-Fragmenten auf, d. h. der frühzeitige Abbruch der Vervielfältigung ist insbesondere hier auf die fehlerhafte Ablesung des Enzyms bei dem Polymerisationsprozeß zurückzuführen.

Daher werden heute häufig DNA-Polymerasen wie z. B. aus Pyrococcus furiosus, sogenannte Pfu-Polymerase verwendet (Lundberg, K.S. et al., Gene 108 (1991) 1-6). Pfu-Polymerase zeichnet sich durch zusätzlich durch eine integrale 3'-(editing)-exonuclease-Aktivität (proofreading activity) aus und ist so imstande, die Mutationsrate pro Cyclus beträchtlich, und zwar um den Faktor von ungefähr 10, zu vermindern. Es hat sich jedoch gezeigt, daß die proofreading-Polymerasen bei der Vervielfältigung von kurzen Sequenzen, d. h. bis ca. 5 kb, an ihre Grenzen stößt. Eine Verbesserung in dieser Hinsicht ist von W. Barnes. in Proc.Natl.Acad.Sci. USA 91 (1994), 2216-2220 bzw. WO 94/26766 beschrieben. Die Verbesserung nach Barnes besteht in dem Einsatz einer Mischung, bestehend aus zwei verschiedenen DNA-Polymerasen, wobei die eine sogenannte proofreading-Activität (wie z. B. Pfu) und die andere, im Überschuß vorliegende DNA-Polymerase keine proofreading-Aktivität (wie z. B. Taq) aufweist. Dadurch werden bei der PCR-Amplifikation von längeren DNA-Sequenzen, d. h. von ca. 10 - 35 kb, jeweils abhängig von der Länge der verwendeten Primer, der Cyclengeschwindigkeit bzw. Cyclenanzahl oder sonstigen Bedingungen, eine höhere Effizienz als auch Ausbeute erzielt. Es hat sich jedoch gezeigt, daß bei kleineren Nukleinsäuresequenzen, d. h. ca. 3 kb oder kleiner, eine Verbesserung hinsichtlich Ausbeute und Spezifitäten nicht erzielt wird.

Der Erfindung lag somit die Aufgabe zugrunde, für die spezifische Vervielfältigung von kurzen Nukleinsäuresequenzen Maßnahmen zur Verfügung zu stellen, durch die die im Stand der Technik beschriebenen Nachteile überwunden werden.

Die Aufgabe wird dadurch gelöst, daß eine Mischung, bestehend aus einer thermophilen DNA-Polymerase mit proofreading-Aktivität und einer thermophilen DNA-Polymerase ohne proofreading-Aktivität zur Vervielfältigung von Nukleinsäuresequenzen mit ca. 5 kb oder kleiner mittels PCR-Reaktion bei einer Elongationstemperatur von mindestens 70 °C verwendet wird. Die zweite DNA-Polymerase liegt dabei im Überschuß vor, vorzugsweise in einer mindestens 10fach höheren Konzentration als das erste Enzym.

Für das erste Enzym kommen beispielsweise DNA-Polymerasen aus Pyrococcus furiosus (Pfu), aus Pyrococcusspezies Thermotoga maritima (Tma), aus Pyrococcus woesii (Pwo), aus Thermococcus literalis (Tli) oder Sulfolobus solfatarimis (Sso) in Betracht. Für das Enzym ohne proofreading-Aktivität haben sich Taq DNA-Polymerase oder entsprechende Analoge wie Klentaq I (N-terminal verkürztes Enzym), das Klenowfragment der DNA-Polymerase I aus E. coli oder andere Polymerasen aus Thermusspecies als geeignet erwiesen. Erfindungsgemäß bevorzugt ist eine Mischung von Pwo und Taq im Verhältnis 1:10.

Darüber hinaus hängen die optimalen Reaktionsbedingungen, wie Inkubationszeit, Temperatur, Pufferbedinungen, Magnesium (Mg²⁺)-Konzentration bzw. die Konzentration der Enzymmischung vom jeweils verwendeten Template/Primer-Paar ab und sollte jeweils individuell bestimmt werden. Entsprechende Vorversuche gehören zu den für den Fachmann üblichen Maßnahmen.

Als optimal für die Mischung hat sich eine Enzymkonzentration im Bereich von 0,5 - 5 U/Testansatz erwiesen, vorzugsweise werden 2,5 U/Testansatz eingesetzt.

Als optimale Mg²⁺-Konzentration hat sich in den meisten Fallen ein Bereich von 0,5 - 5 mM, vorzugsweise ca. 1,5 mM, erwiesen. In der Regel wird MgCl₂ verwendet. Es wurde zudem festgestelt, daß das erfindungsgemäße Verfahren zur Amplifikation kurzer Fragmente gegenüber einer mit Taq-Polymerase durchgeführten PCR-Reaktion den Vorteil hat, daß es eine weitaus geringere Empfindlichkeit gegenüber hohen Mg²⁺-Konzentrationen aufweist.

Darüber hinaus können übliche im Bereich von pH 7,0 - 9,5 puffernde Substanzen, wie beispielsweise Hepes, Phosphat oder Tris-HCl als Puffer für die Reaktion verwendet werden.

Erfindungsgemäß hat sich eine Pufferkonzentration, beispielsweise von Tris-HCl, im Bereich von ca. 5 - 100 mM, vorzugsweise von ca. 50 mM, sowie die Anwesenheit bestimmter Salze als vorteilhaft erwiesen. Bei letzteren haben sich Ammoniumsulfat (AS), und zwar in einer Konzentration von ca. 18 - 30 mM, bevorzugt von 20 - 24 mM sowie Kaliumchlorid (KCl) in einer Konzentration von ca. 30 - 50 mM als besonders vorteilhaft erwiesen. Als pH-Wert für die Amplifikation von kurzen Fragmenten hat sich ein pH-Wert von ca. 8,9 als besonders geeignet erwiesen.

Darüber hinaus kann die PCR-Reaktion erfindungsgemäß durch den Zusatz weiterer Substanzen wie beispielsweise Rinderserumalbumin in einer Konzentration von bis zu 100 µg/ml, SH-Reagenzien wie Dithiothreitol oder ein Detergenz wie beispielsweise Tween®20, Nonidet® oder Glycerol oder DMSO in üblichen Konzentrationen weiter verbessert werden.

Für die Amplifikation von kurzen Nukleinsäuresequenzen, d. h. die annähernd 3 kb oder kürzer sind, hat sich insbesondere eine Temperatur für den Verlängerungsschritt von ca. 70 - 75 °C, vorzugsweise von 72 °C als vorteilhaft erwiesen. Die Elongationszeit beträgt zwischen ca. 30 Sekunden und 8 Minuten und hängt stark von der Länge des zu amplifizierenden Fragments ab. Für DNA-Fragmente bis zu 0,75 kb haben hier insbesondere 45 Sekunden, für Fragmente von ca. 1,5 kb ca. eine Minute und ca. zwei Minuten für DNA-Fragmente in der Größenordnung von 3 kb.

Ein für die DNA-Amplifikation verwendetes Reagenz besteht vorteilhafterweise aus zwei Einzelmischungen. Die erste Mischung enthält die Template-DNA, wie z. B. humane genomische DNA in einem Konzentrationsbereich von ca 1 - 15 µg/ml mit sogenannten upstream- und downstream-Primer (vorzugsweise je ca. 300 nM) und sämtliche für die DNA-Kettenverlängerung erforderlichen Nukleotide, wie dATP, dCTP, dGTP und dTTP. Für die einzelnen Nukleotide hat sich jeweils eine Konzentration von 150 - 300 µM als vorteilhaft erwiesen.

Die zweite Mischung enthält im wesentlichen den für die PCR-Reaktion erforderlichen Puffer und die erfindungsgemäße Enzymmischung in einer entsprechend höher konzentrierten Form, so daß sich nach Vermischung mit der ersten Mischung die eingangs beschriebenen vorteilhaften Konzentrationen ergeben. Als besonders geeignet haben sich für die Amplifikation von kurzen Fragmenten Reaktionsansätze von 100 µl oder 50 µl erwiesen. Nach Zusammenmischung der Einzelmischungen wird die Probe in ein entsprechendes Thermocycler-Gerät eingebracht und zunächst zur Trennung des Doppelstranges des jeweiligen DNA-Fragments denaturiert (bei 94 °C, 2 Minuten). Daran schließen sich die einzelnen Cyclen der PCR, wobei sich bei dem Einsatz kurzer DNA-Fragmente (bis 3 kb) eine Temperatur von 70 - 74 °C als besonders vorteilhaft erwiesen hat. Des weiteren ist bei der erfindungsgemäßen Anwendung als vorteilhaft herauszustellen, daß die einzelnen Elongationsschritte bedeutend kürzer sind und somit ein nicht unwesentlicher Zeitgewinn der erfindungsgemäßen DNA-Amplifikation gegenüber bekannten Verfahren festzustellen ist.

Das erfindungsgemäße Verfahren kann darüber hinaus vorteilhaft für die Markierung von kurzen DNA-Fragmenten eingesetzt werden. Wie bei der Markierung von DNA-Fragmenten bei der PCR-Reaktion prinzipiell vorzugehen ist, ist dem Fachmann bekannt (z. B. EP 0 420 260; WO 90/11373).

Vorteilhaft ist erfindungsgemäß, daß die Enzymmischung, bevorzugt bestehend aus Pwo und Taq im Verhältnis von ca. 1:10, unter den spezifischen Bedingungen besonders gut modifizierte Nukleotide wie Digoxigenin-dUTP, Biotin-dUTP oder Fluoreszein-dUTP akzeptiert. Die Konzentrationen für diese modifizierten Nukleotide liegen optimalerweise bei jeweils ungefähr 50 µM, die von dTTP bei ca. 150 µM, wenn die Probes anschließend über Southern Blot nachgewiesen werden. Für die Analyse mittels ELISA hat sich eine Konzentration von modifiziertem dUTP von 10 µM und der entsprechenden Menge an dTTP als vorteilhaft erwiesen. Für den Fall, daß als modifiziertes Nukleotid Biotin-dUTP eingesetzt wird, hat sich eine höhere Konzentration an Magnesium, und zwar bis zu 4 mM MgCl₂ als besonders vorteilhaft erwiesen.

### Abkürzungsverzeichnis

- Bio-dUTP: Biotin16-2'-desoxy-uridin-5'-triphosphat
- bp: Basenpaare
- C: Celsius
- CF: Cystische Fibrose
- dATP: 2'-Desoxy-adenin-5'-triphosphat
- dCTP: 2'-Desoxy-cytidin-5'-triphosphat
- DIG-dUTP: Digoxigenin 11-2'-desoxy-uridin-5'-triphosphat
- dGTP: 2'-Desoxy-guanin-5'-triphosphat
- dNTP: 2'-Desoxy-nukleosid-5'-triphosphat
- DTT: 1,4-Dithiothreit
- dTTP: 2'-Desoxy-thymidin-5'-triphosphat
- EDTA: (Ethylendinitrilo)tetraessigsäure
- HCl: Salzsäure
- kb: Kilobasen
- KCl: Kaliumchlorid
- Min.: Minute
- mM: milimolar
- µM: mykromolar
- ng: Nanogramm
- (NH₄)₂SO₄: Ammoniumsulfat
- p53: Protein53
- PCR: Polymerase Ketten Reaktion
- Pwo: DNA Polymerase aus Pyrococcus woesii
- Sek.: Sekunde
- Taq: DNA Polymerase aus Thermus aquaticus
- tPA: tissue Plasminogen Activator
- Tris: 2-Amino-2(hydroxymethyl)-1,3-propandiol
- U: Units

### Primerverzeichnis

- Collagen Primer 1:: 5' - TAA AGG GTC ACC GTG GCT TC - 3'
- Collagen Primer 2:: 5' - CGA ACC ACA TTG GCA TCA TC - 3'
- p 53 Primer 1:: 5' - TGG AAA CTT TCC ACT TGA T - 3'
- p 53 Primer 2:: 5' - GTC CCA AGC AAT GGA TCA T - 3'
- FKBP Primer 1:: 5' - GGA ATT CTA TGG GAG TGC AGG TGG AA - 3'
- FKBP Primer 2:: 5' - GCG GAT CCA AGC TTT CAT TCC AGT TTT AGA AGC TC - 3'
- CF Primer 1:: 5' - GCT GCA TCA TAT AAG TTG CC - 3'
- CF Primer 2:: 5' - AAG GCT ACA CTG TTA ATT TT - 3'
- p 53 Primer 3:: 5' - GTC CCA AGC AAT GGA TGA T - 3'
- p 53 Primer 4:: 5' - TGG AAA CTT TCC ACT TGA T - 3'
- tPA Primer 7:: 5' - GGA AGT ACA GCT CAG AGT TCT GCA GCA CCC CTG C - 3'
- tPA Primer 10:: 5' - GAT GCG AAA CTG AGG CTG GCT GTA CTG TCT C - 3'
- tPA Primer 13:: 5' - TGT CTC CAG CAC ACA GCA TGT TGT CGG TGA C - 3'
- tPA Primer 14:: 5' - CAA AGT CAT GCG GCC ATC GTT CAG ACA CAC C - 3'
- tPA Primer 1:: 5' - AGA CAG TAC AGC CAG CCT CA - 3'
- tPA Primer 2:: 5' - GAC TTC AAA TTT CTG CTC CTC - 3'

### Legende zu den Abbildungen:

### Abbildung 1

Amplifikation eines 1.1 kb Fragments aus dem Collagen Gen aus humaner DNA, mit Taq DNA Polymerase (Stand der Technik) und Taq/Pwo-Mischung (erfindungsgemäß) in Abhängigkeit von der Template-Menge.

Als Template-Variation wurden jeweils 250 ng, 100 ng, 50 ng, 10 ng und 1 ng genomische DNA verwendet. Die Enzymmischung zeigt bei jeder Konzentration deutlich mehr PCR Produkt, selbst 1 ng wurde effizient amplifiziert. Mit Taq DNA Polymerase sind gerade noch 10 ng Ausgangs-DNA nachweisbar. Dies zeigt, daß die Enzymmischung in diesem Beispiel ca. 50 mal effizienter amplifiziert als Taq Polymerase.

### Abbildung 2

Amplifikation eines 307 bp Fragments aus dem P 53 Gen (aus humaner DNA) mit Taq DNA Polymerase (Stand der Technik) und Taq/Pwo-Mischung (erfindungsgemäß) in Abhängigkeit von der Mg-Ionen-Konzentration. Als Mg-Ionen-Konzentrationen wurden 1.5; 1.75; 2.0; 2.5; 3.0; 5.0 mM eingesetzt.

Die Enzymmischung zeigt über alle Mg-Konzentrationen eine effizientere Amplifikation der Ausgangs-DNA. Mit Taq DNA Polymerase wird nur ein deutlich sichtbares Produkt mit 1.5 mM Magnesium erzielt. Dies zeigt, daß die Enzymmischung deutlich weniger anfällig gegenüber Mg-Einflüssen ist.

### Abbildung 3

Amplifikation von 324 bp, 950 bp, 2.9 kb, 4.8 kb, 6.5 kb und 9.3 kb Fragmenten aus humaner, genomischer DNA mit Taq/Pwo-Enzymmischung (erfindungsgemäß) und Taq DNA Polymerase (Stand der Technik).

Mit der Enzymmischung können über den ganzen Bereich PCR-Produkte erzielt werden. Mit Taq-Polymerase ist es nur möglich bis 3 kb PCR-Produkte zu amplifizieren. In allen Fällen zeigt die Enzymmischung deutlich mehr Produkt als Taq-Polymerase.

### Abbildung 4

Amplifikation und Markierung eines 375 bp Fragmentes aus dem tPA Gen aus humaner DNA mit Pwo-DNA-Polymerase, Taq-DNA-Polymerase und der Enzymmischung in Abhängigkeit von verschiedenen Konzentrationen der modifizierten Nukleotiden DIG-dUTP und Bio-dUTP. Sowohl der Einbau von Digoxigenin-dUTP als auch von Biotin-dUTP, unabhängig von der Konzentration des modifizierten Nukleotides, wird mit der Enzymmischung am besten vollzogen. Dies bezieht sich sowohl auf die Effizienz (Produktmenge) als auch auf die Dichte der Markierung (Schrift der PCR-Produkte zu höherem Molekulargewicht, was einen höheren Einbau von modifiziertem dUTP entspricht). Pwo-Polymerase zeigt im Ethidium-Bromid Gel keine spezifischen PCR-Produkte, nur unspezifischen Hintergrund. Taq-DNA-Polymerase zeigt nur teilweise eine spezifische Amplifikation, auch hier ist ein hoher unspezifischer Hintergrund sichtbar.

Die folgenden Beispiele verdeutlichen die Erfindung weiter:

### Beispiel 1:

In den gezeigten Beispielen wurde als Taq/ Pwo Enzymmischung (erfindungsgemäß) die thermostabilen Polymerasen von Thermus aquaticus (Taq) und Pyrococcus woesii (Pwo) verwendet. Das Mischungsverhältnis der beiden Polymerasen war 10:1 (Taq : Pwo) nach Aktivität (Units). Eine typische Enzymmischung war 3.14 U Taq Polymerase + 0.35 U Pwo Polymerase pro µl. Die Enzymmischung wurde in Lagerpuffer (20 mM Tris/HCl, pH 7.5 (20^{o}C), 100 mM KCl, 1 mM DTT, 0.1 mM EDTA, 0.5% Tween20, 0.5% Nonidet P40, 50 % Glycerin) bei -20^{o}C gelagert.

Die Polymerase-Ketten-Reaktion (PCR) wurde im 100 µl Volumen durchgeführt. Die Reaktionbedingungen für die Enzymmischung war wie folgt:

| | |
|---|---|
| PCR-Puffer: | 22 mM (NH₄)₂SO₄; 50 mM Tris/HCl(20^{o}C) |
| MgCl₂ | 1.5 mM |
| Enzymmischung | 2.6 U |
| dNTP | 200 µM (jeweils) |
| Collagen Primer 1 | 300 nM |
| Collagen Primer 2 | 300 nM |

Als Template wurde genomische DNA (human) mit 5 verschiedenen Konzentrationen verwendet 250 ng, 100 ng, 50 ng, 10 ng und 1 ng.

Parallel zu den Ansätzen mit der Enzymmischung wurden PCR Reaktionen mit Taq DNA Polymerase durchgeführt. Der einzige Unterschied zu dem vorher beschriebenen Ansatz besteht im PCR Puffer, hier wurde der Taq Puffer verwendet (50 mM KCl, 10 mMTris/HCl pH 8.3 (20^{o}C)) sowie 2.5 U Taq DNA Polymerase.

Die Amplifikationen wurden in einem Perkin Elmer GenAmp 9600 Thermocycler mit folgendem Cycleprogramm durchgeführt:

| | |
|---|---|
| 1x | Denaturierung für 2 Min. bei 94^{o}C |
| 10x | Denaturierung für 15 Sek. bei 94^{o}C |
| | Annealing für 30 Sek. bei 60^{o}C |
| | Elongation für 45 Sek. bei 72^{o}C |
| 20x | Denaturierung für 15 Sek. bei 94^{o}C |
| | Annealing für 30 Sek. bei 60^{o}C |
| | Elongation für 45 Sek. bei 72^{o}C + Cycle Verlängerung |
| | für 20 Sek. für jeden weiteren Cycle |
| 1x | 7 Min. bei 72^{o}C |

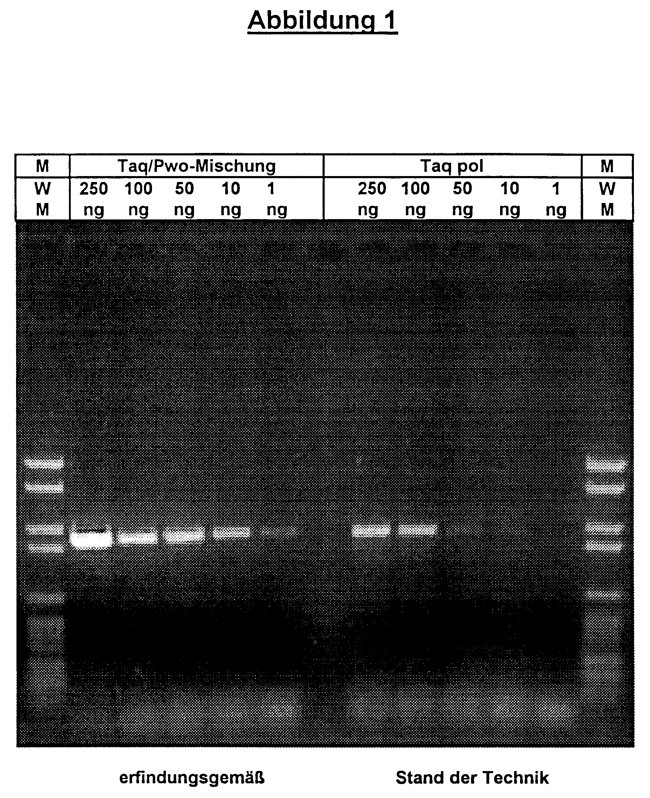

25 µl des erhaltenen PCR Produktes wurde auf einem 1%igen Agarose-Gel analysiert. Abbildung 1 zeigt, die Amplifikation eines 1.1 kb Fragments aus dem Collagen Gen aus humaner DNA, mit Taq DNA Polymerase (Stand der Technik) und Taq/Pwo-Mischung (erfindungsgemäß) in Abhängigkeit von der Template-Menge.

Die Enzymmischung zeigt bei jeder Template Konzentration deutlich mehr PCR Produkt, selbst 1 ng wurde effizient amplifiziert. Mit Taq DNA Polymerase sind gerade noch 10 ng Ausgangs-DNA nachweisbar. Dies zeigt, daß die Enzymmischung in diesem Beispiel ca. 50 mal effizienter amplifiziert als Taq DNA Polymerase.

### Beispiel 2:

Die Amplifikationen mit der Enzymmischung als auch mit Taq DNA Polymerase wurde identisch zu Beispiel 1 durchgeführt mit folgenden Änderungen:

Template DNA (human-genomische DNA) war 250 ng. Als Primer wurden p53 primer 1 als forward Primer und p53 Primer 2 als reverse Primer verwendet.

Die Magnesium Konzentration wurde wie folgt verwendet: 1.5 mM, 1.75 mM, 2.0 mM, 2.5 mM, 3.0 mM und 5.0 mM. Das Cycle-Programm und die Analytik der PCR Produkte war wie in Beispiel 1.

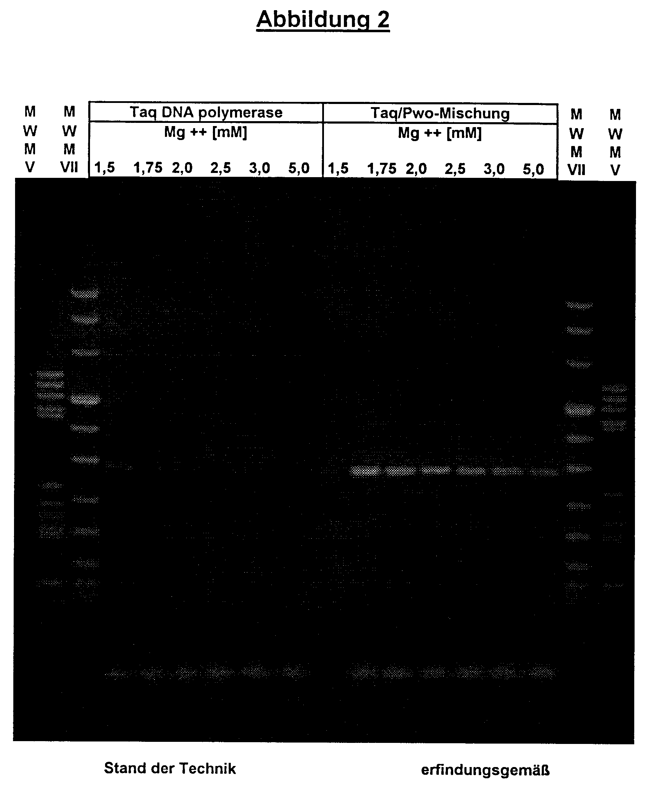

Abbildung 2 zeigt die Amplifikation eines 307 bp Fragments aus dem p53 Gen mit Taq DNA Polymerase (Stand der Technik) und Taq/Pwo-Mischung (erfindungsgemäß) in Abhängigkeit von der Mg-Ionen-Konzentration.

Die Enzymmischung zeigt über alle Mg-Konzentrationen eine effizientere Amplifikation der Ausgangs-DNA. Mit Taq DNA Polymerase wird nur ein deutlich sichtbares Produkt mit 1.5 mM Magnesium erzielt. Dies zeigt, daß die Enzymmischung deutlich weniger anfällig gegenüber Mg-Einflüssen ist.

### Beispiel 3:

Eine Reihe von verschiedenen PCR Fragmenten wurden aus 250 ng genomischer DNA amplifiziert. Die Bedingungen für die PCR Reaktion sowie das Cycle Programm waren wie in Beispiel 1 beschrieben mit folgenden Änderungen:

Folgende Primerpaare und Bedingungen wurden verwendet:
1) FKBP Primer 1/ FKBP Primer 2 (324 bp)
2) CF Primer 1/ CF Primer 2 (950 bp)
3) p53 Primer 3/ p53 Primer 4 (2.9 kb); Elongationszeit 2 Min.
4) tPA Primer 7/ tPA Primer 10 (4.8 kb); Elongationszeit 4 Min.
5) tPA Primer 7/ tPA Primer 13 (6.5 kb); Elongationszeit 5 Min.
6) tPA Primer 7/ tPA Primer 14 (9.4 kb); Elongationszeit 8 Min.

Für 4) -6) wurden 50 µl Reaktionsansätze verwendet und die Elongationstemperatur wurde mit 65^{o}C gewählt.

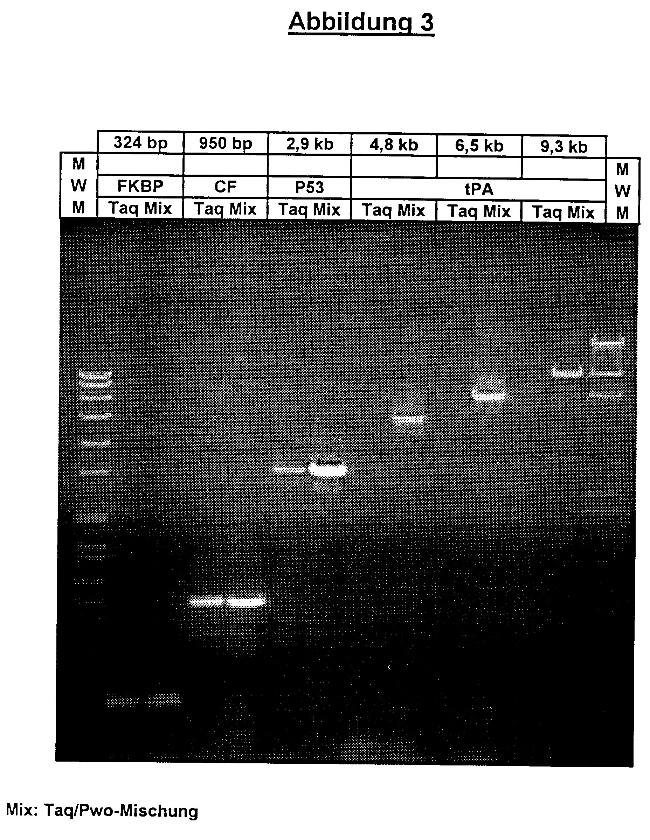

Abbildung 3 zeigt die Amplifikation von 324 bp, 950 bp, 2.9 kb, 4.8 kb, 6.5 kb und 9.3 kb Fragmenten aus humaner, genomischer DNA mit Taq/Pwo-Enzymmischung (erfindungsgemäß) und Taq DNA Polymerase (Stand der Technik).

Mit der Enzymmischung können über den ganzen Bereich PCR-Produkte erzielt werden. Mit Taq-Polymerase ist es nur möglich, bis 3 kb PCR-Produkte zu amplifizieren. In allen Fällen zeigt die Enzymmischung deutlich mehr Produkt als Taq-Polymerase.

### Beispiel 4:

Die Amplifikationbedingungen als auch die Cyclebedingungen waren wie im Beispiel 1 aufgeführt, mit folgenden Änderungen:

Es wurden die tPA Primer 1 und tPA Primer 2 verwendet. Die Menge an humaner DNA war 100 ng.

Für Pwo wurden folgende PCR Puffer Bedingungen gewählt: 10 mM Tris/HCl pH 8.85; 25 mM KCl, 5 mM (NH₄)₂SO₄). Die Magnesium Sulfat Konzentration war 3.5 mM. Es wurden 2.5 U Pwo DNA Polymerase pro Reaktion eingesetzt.

Es wurden folgende dNTP Mischungen für die Experimente verwendet:
**1** je 200 µM dGTP, dCTP, dATP, dTTP
**2** je 200 µM dGTP, dCTP, dATP, dTTP; 100 µM dTTP, 100 µM Biotin-dUTP
**3** je 200 µM dGTP, dCTP, dATP, dTTP; 134 µM dTTP, 66 µM Biotin-dUTP
**4** je 200 µM dGTP, dCTP, dATP, dTTP; 180 µM dTTP, 20 µM Biotin-dUTP
**5** je 200 µM dGTP, dCTP, dATP, dTTP; 134 µM dTTP, 66 µM Digoxigenin-dUTP
**6** je 200 µM dGTP, dCTP, dATP, dTTP; 190 µM dTTP, 10 µM Digoxigenin-dUTP

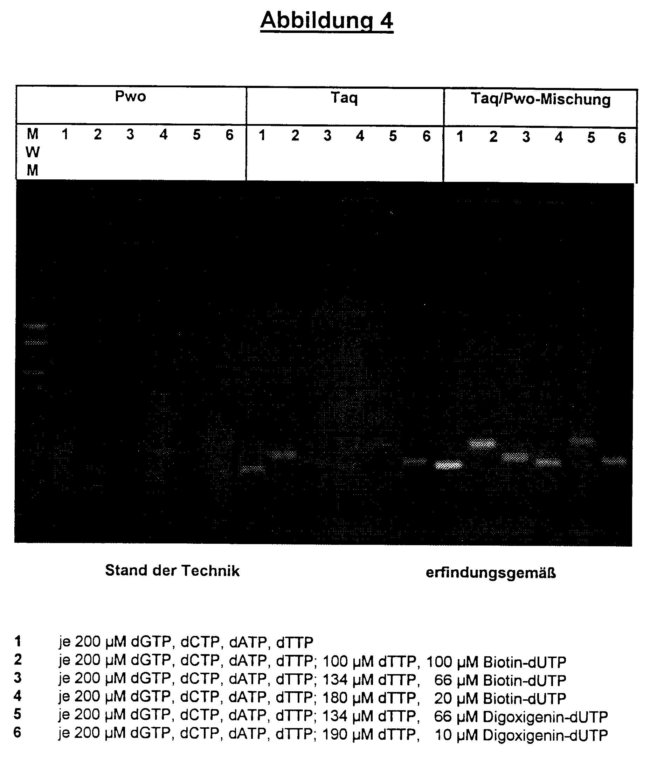

Abbildung 4 zeigt die Amplifikation und Markierung eines 375 bp Fragmentes aus dem tPA Gen aus humaner DNA mit Pwo-DNA-Polymerase, Taq-DNA-Polymerase und der Enzymmischung in Abhängigkeit von verschiedenen Konzentrationen der modifizierten Nukleotiden DIG-dUTP und Bio-dUTP. Sowohl der Einbau von Digoxigenin-dUTP als auch von Biotin-dUTP, unabhängig von der Konzentration des modifizierten Nukleotides, wird mit der Enzymmischung am besten vollzogen. Dies bezieht sich sowohl auf die Effizienz (Produktmenge) als auch auf die Dichte der Markierung (Schrift der PCR-Produkte zu höherem Molekulargewicht, was einen höheren Einbau von modifiziertem dUTP entspricht). Pwo-Polymerase zeigt im Ethidium-Bromid Gel keine spezifischen PCR-Produkte, nur unspezifischen Hintergrund. Taq-DNA-Polymerase zeigt nur teilweise eine spezifische Amplifikation, auch hier ist ein hoher unspezifischer Hintergrund sichtbar.

## Patentansprüche

1. Verfahren zur spezifischen Vervielfältigung von kurzen ein- oder doppelsträngigen DNA-Fragmenten in Gegenwart mindestens eines geeigneten Primer-Paares, einer zwischen pH 7,0 und 9,5 puffernden Substanz und einer Enzymmischung, dadurch gekennzeichnet, daß die Enzymmischung aus einer thermophilen DNA-Polymerase mit proofreading-Aktivität und einer thermophilen DNA-Polymerase ohne proofreading-Aktivität besteht und nach gegebenenfalls stattgefundenem Denaturierungsschritt zur Spaltung von doppelsträngigen DNA-Fragmenten der Elongationsschritt zwischen 30 Sekunden und 4 Minuten bei mindestens 70 °C durchgeführt wird.

2. Verfahren nach Anspruch 1, dadurch gekennzeichnet, daß die Enzymmischung eine DNA-Polymerase ohne proofreading-Aktivität im mindestens 10fachen Überschuß aufweist.

3. Verfahren nach Anspruch 1 oder 2, dadurch gekennzeichnet, daß die Enzymmischung DNA-Polymerasen aus Pyrococcus woesii und aus Thermus aquaticus enthaltend die Enzymkonzentration 0,5 U bis 5,0 U in der Reaktionsmischung beträgt.

4. Verfahren nach einem der vorangegangenen Ansprüche, dadurch gekennzeichnet, daß Magnesiumionen in einem Konzentrationsbereich von 0,5 bis 5,0 mM zugegen sind.

5. Verfahren nach einem der vorangegangenen Ansprüche, dadurch gekennzeichnet, daß ungefähr 18 bis 30 mM Ammoniumsulfat und ungefähr 30 bis 50 mM Kaliumchlorid in der Reaktionsmischung, die einen Ausgangs-pH-Wert von ca. 8,9 aufweist, vorhanden sind.

6. Verfahren nach einem der vorangegangenen Ansprüche, dadurch gekennzeichnet, daß die Elongationstemperatur ca. 72 °C beträgt.

7. Enzymmischung bestehend aus einer thermophilen DNA-Polymerase mit proofreading-Aktivität und einer thermophilen DNA-Polymerase ohne proofreading-Aktivität im Verhältnis 1 : 10 und die Konzentration der Enzyme so angelegt ist, daß die Endkonzentration 2,0 bis 3,0 U/Volumen Reaktionsmischung beträgt.

8. Enzymmischung nach Anspruch 7, dadurch gekennzeichnet, daß DNA-Polymerase aus Pyrococcus woesii und Thermus aquaticus sowie Magnesiumionen in einer Konzentration von 1,0 bis 5,0 mM zugegen sind.

9. Verwendung der Enzymmischung gemäß Anspruch 7 oder 8 zur Vervielfältigung kurzer DNA-Fragmente, insbesondere bis ca. 3 kb.

10. Verwendung der Enzymmischung gemäß Anspruch 7 oder 8 zur Vervielfältigung kurzer DNA-Fragmente und deren Markierung mit modifizierten Nukleotiden.

11. Verwendung nach Anspruch 10, dadurch gekennzeichnet, daß als modifiziertes Nukleotid Biotin-dUTP in Gegenwart einer Magnesiumkonzentration von ca. 4 mM eingesetzt wird.
